# EUROPEAN PATENT APPLICATION

(11) **EP 4 115 913 A1**
(43) Date of publication of application: **11.01.2023**
(21) Application number: 22165624.2
(22) Date of filing: 30.03.2022
(51) Int. Cl.: A61L 9/03, C11C 5/00, F21V 35/00, H05B 6/02

(54) **SCENTED CANDLE AND SCENTED CANDLE DEVICE**

(30) Priority: 05.07.2021 TW 110207841 U
(71) Applicant: Chang, I-Min, New Taipei City 22441 (TW); Chang, Tzu-Wen, New Taipei City 22441 (TW); Chen, Pei-Lun, New Taipei City 22441 (TW)
(72) Inventor: Chang, I-Min, New Taipei City 22441 (TW); Chang, Tzu-Wen, New Taipei City 22441 (TW); Chen, Pei-Lun, New Taipei City 22441 (TW)
(74) Representative: Regimbeau

(57) **Abstract**

A scented candle device includes an electromagnetic base (2) having a casing (21) that defines a base space (22) and that has a top surface (211), and an electromagnetic unit (23) disposed in the base space (22) for generating a magnetic field. An electromagnetically heated scented candle (3) is disposed on the top surface (211), and includes a container (31) defining a receiving space (313), and a scented candle body (32) received in the receiving space (313). A magnetically conductive heating member (4) is disposed between the top surface (211) and the container (31), and generates heat energy after interacting with the magnetic field of the electromagnetic unit (23) for heating the scented candle body (32).

## Description

The disclosure relates to a candle and a candle device, and more particularly to a scented candle and a scented candle device.

A scented candle can be used as an ornament or a fragrance diffuser to create an ambience, and is welcomed by consumers.

At present, most scented candles on the market need to be lit with an open flame. However, the open flame can easily cause burns or even fire due to human negligence, thereby resulting in losses to customers. Further, because the temperature of the open flame is not easy to control, the conventional scented candle is unevenly heated, and is unable to effectively and uniformly volatilize the fragrance thereof into the environment.

Therefore, an object of the present disclosure is to provide an electromagnetically heated scented candle that can alleviate at least one of the drawbacks of the prior art.

According to one aspect of this disclosure, an electromagnetically heated scented candle includes a container and a scented candle body. The container includes a bottom wall and a surrounding wall extending upwardly from a periphery of the bottom wall. The bottom wall and the surrounding wall cooperatively defines a receiving space. The bottom wall is magnetically conductive. The scented candle body is received in the receiving space.

Another object of this disclosure is to provide a scented candle device.

According to another aspect of this disclosure, a scented candle device includes an electromagnetic base, an electromagnetically heated scented candle, and a magnetically conductive heating member.

The electromagnetic base includes a casing that defines a base space and that has a top surface, and an electromagnetic unit disposed in the base space for generating a magnetic field. The electromagnetically heated scented candle is disposed on the top surface, and includes a container and a scented candle body. The container includes a bottom wall contactable with the top surface of the casing, and a surrounding wall extending upwardly from a periphery of the bottom wall. The bottom wall and the surrounding wall cooperatively define a receiving space. The magnetically conductive heating member is disposed between the top surface of the casing and the container, and generates heat energy after interacting with the magnetic field of the electromagnetic unit for heating the scented candle body.

Other features and advantages of the disclosure will become apparent in the following detailed description of the embodiments with reference to the accompanying drawings, of which:
Figure 1 is a schematic view of a scented candle device according to a first embodiment of the present disclosure;
Figure 2 is a schematic view of a scented candle device according to a second embodiment of the present disclosure;
Figure 3 is a schematic view of a scented candle device according to a third embodiment of the present disclosure; and
Figure 4 is a schematic view of a scented candle device according to a fourth embodiment of the present disclosure.

Before the disclosure is described in greater detail, it should be noted that where considered appropriate, reference numerals or terminal portions of reference numerals have been repeated among the figures to indicate corresponding or analogous elements, which may optionally have similar characteristics.

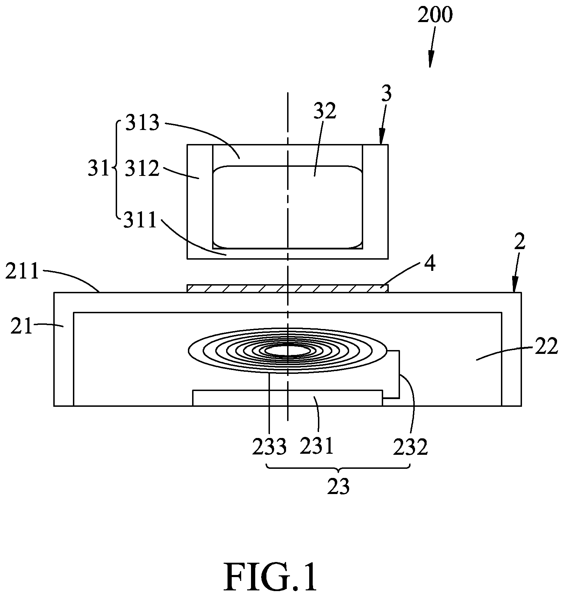

Referring to Figure 1, a scented candle device 200 according to a first embodiment of the present disclosure is heated by electromagnetic heating method, and includes an electromagnetic base 2, an electromagnetically heated scented candle 3, and a magnetically conductive heating member 4.

The electromagnetic base 2 includes a casing 21 and an electromagnetic unit 23. The casing 21 defines a base space 22 and has a top surface 211.

The electromagnetic unit 23 is disposed in the base space 22 for generating a magnetic field, and includes a power supply 231, a control circuit 232 electrically connected to the power supply 231, and a loading coil 233 electrically connected to the control circuit 232. The loading coil 233 is capable of generating the magnetic field by current supplied by the power supply 231. The control circuit 232 is used to adjust the parameters (e.g., current magnitude, direction or AC frequency, etc.) of the current supplied by the power supply 231, thereby controlling the magnitude of the magnetic field generated by the loading coil 233. In this embodiment, the power supply 231 can be a wireless power supply, a USB power supply, or a conventional household AC power supply, but is not limited hereto. As long as the current can be introduced to the loading coil 233 through the control circuit 232, any type of power supply is acceptable.

The electromagnetically heated scented candle 3 is disposed on the top surface 211 of the casing 21, and includes a container 31 and a scented candle body 32. The container 31 includes a bottom wall 311 contactable with the top surface 211 of the casing 21, and a surrounding wall 312 extending upwardly from a periphery of the bottom wall 311. The bottom wall 311 and the surrounding wall 312 cooperatively define a receiving space 313. The scented candle body 32 is wickless and is received in the receiving space 313. The bottom wall 311 has a thickness equal to or different from that of the surrounding wall 312. To facilitate heat conduction, at least the bottom wall 311 or the container 13 can be made of a material with good thermal conductivity. In this embodiment, the bottom wall 311 is made of a metal material, but not limited thereto.

The magnetically conductive heating member 4 is disposed between the top surface 211 of the casing 21 and the bottom wall 311 of the container 31. The magnetically conductive heating member 4 generates heat energy after interacting with the magnetic field of the electromagnetic unit 23 for heating the scented candle body 32. In this embodiment, the magnetically conductive member 4 is exemplified as a magnetic spacer. The magnetically conductive member 4 may be made of a stainless steel or ferrite magnetic material. A surface area of the magnetically conductive member 4 is substantially same as a surface area of the bottom wall 311 of the container 31 of the electromagnetically heated scented candle 3. However, in actual practice, the surface area of the magnetically conductive member 4 may be greater or smaller than the surface area of the bottom wall 311 of the container 31. As long as the bottom wall 311 can be uniformly heated, there is no particular limitation to the size of the surface area of the magnetically conductive member 4.

To use the scented candle device 200, the magnetically conductive member 4 is first disposed on the top surface 211 of the casing 21, after which the electromagnetically heated scented candle 3 is placed on top thereof, so that the magnetically conductive member 4 is directly in contact with the bottom wall 311 of the container 31 of the electromagnetically heated scented candle 3. Next, the power supply 231 is activated, and the current supplied by the power supply 231 is introduced to the loading coil 233 through the control circuit 232, so that the loading coil 233 can generate an alternating magnetic field, and an eddy current is generated by the electromagnetically conductive member 4 disposed adjacent to the loading coil 233 and the alternating magnetic field due to electromagnetic induction. Based on the impedance of the electromagnetically conductive member 4 itself, a heating effect of current occurs to generate heat. Then, the heat is transferred to the scented candle body 32 through the bottom wall 311 to achieve the effect of heating the scented candle body 32 and to diffuse the fragrance thereof into the environment.

Because the scented candle device 200 of this disclosure uses the heat generated by the electromagnetic unit 23 and the electromagnetically conductive member 4 due to electromagnetic induction to heat the scented candle body 32, and because the magnitude of the magnetic field generated by the loading coil 233 can be controlled by the control circuit 232, the temperature for heating the scented candle body 32 can be adjusted according to the requirement. Further, in comparison with the conventional scented candle that requires the open flame to lit it so that it is unevenly heated, because the electromagnetically conductive member 4 directly contacts the bottom wall 311 of the container 31 of the electromagnetically heated scented candle 3, the heat energy generated by the electromagnetically conductive member 4 can be evenly distributed to the bottom wall 311 and transferred to the scented candle body 32, so that the scented candle body 32 can be evenly heated, and the fragrance thereof can be uniformly diffused into the environment.

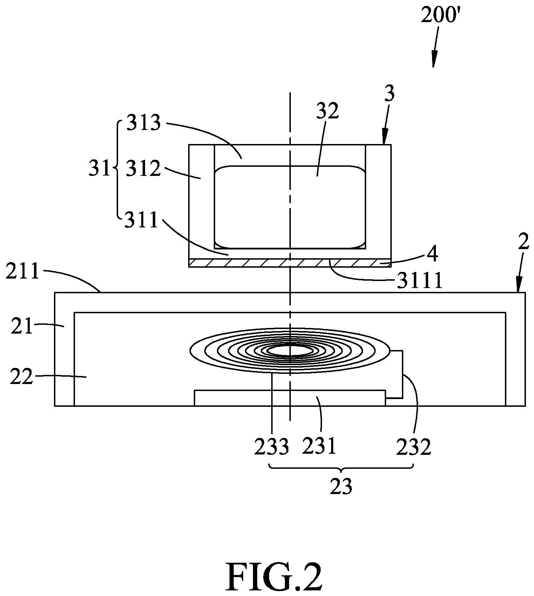

Figure 2 illustrates a scented candle device 200' according to a second embodiment of the present disclosure. The difference between the first and second embodiments resides in that the electromagnetically conductive member 4 of the second embodiment is connected to the bottom wall 311 of the container 31 of the electromagnetically heated scented candle 3.

Specifically, the bottom wall 311 of the container 31 has a bottom surface 3111. The magnetically conductive heating member 4 is connected to the bottom surface 3111 and is located outside of the receiving space 313. A surface area of the bottom surface 3111 may be equal to or different from that of the magnetically conductive heating member 4. It should be noted that the magnetically conductive heating member 4 can be fixed to or removably connected to the bottom surface 3111 through different methods, such as bonding, embedding, snapping, etc. Since the detailed structures and implementations of these joining methods are well known to those in the relevant technical field, a detailed description thereof is omitted herein for the sake of brevity.

In this embodiment, the electromagnetically heated scented candle 3 is disposed on the top surface 211 of the casing 21 of the electromagnetic base 2 through the magnetically conductive heating member 4 that is connected to the bottom wall 311 of the container 31. Similarly, the heat energy generated by the electromagnetically conductive member 4 and the electromagnetic unit 23 due to electromagnetic induction is used to heat the scented candle body 32 of the electromagnetically heated scented candle 3.

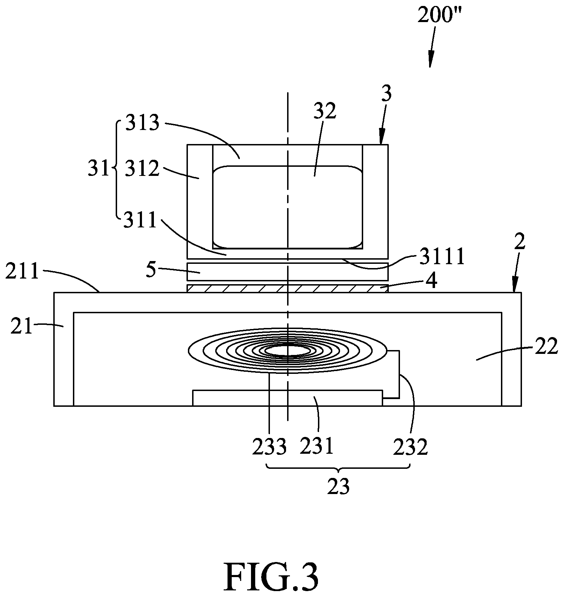

Referring to Figure 3, a scented candle device 200" according to a third embodiment of the present disclosure is identical to the first embodiment, and differs in that, in the third embodiment, the scented candle device 200" further includes a thermally conductive bonding layer 5 disposed between the bottom surface 3111 of the bottom wall 311 of the container 31 and the magnetically conductive heating member 4. The thermally conductive bonding layer 5 is deformable, and is made of a material selected from the group consisting of silicone, soft plastic, and nanomaterials. The thermally conductive bonding layer 5 can enhance adhesion between the metal bottom wall 311 of the container 31 and the magnetically conductive heating member 4 so as to avoid a gap at a junction of the bottom wall 311 and the magnetically conductive heating member 4 when they directly contact each other. Hence, heat loss can be reduced, and the heat can be transferred to the scented candle body 32 more efficiently.

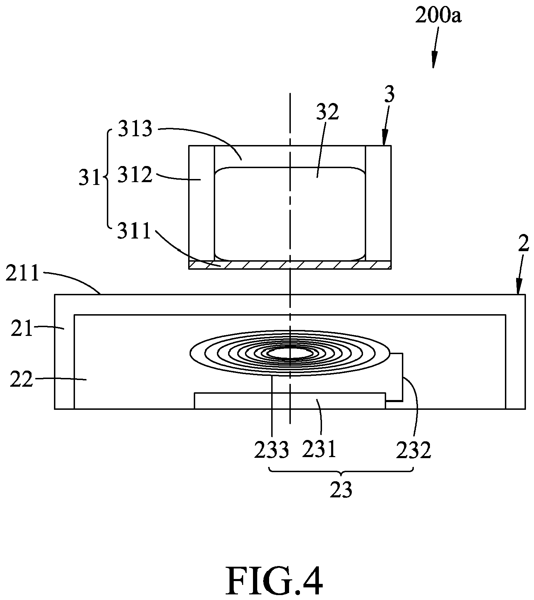

Referring to Figure 4, a scented candle device (200a) according to a fourth embodiment of the present disclosure is identical to the first embodiment, and differs in that, in the fourth embodiment, the bottom wall 311 of the container 31 of the electromagnetically heated scented candle 3 serves as a magnetically conductive heating member. That is, at least the bottom wall 311 of the container 31 is made of a magnetic material, or the entire container 31 is made of the magnetic material. In this way, the bottom wall 311 can function similar to the magnetically conductive heating member 4 (see FIG. 1) described in the first embodiment, and can interact with the magnetic field of the electromagnetic unit 23 to generate heat for heating the scented candle body 32.

In the description above, for the purposes of explanation, numerous specific details have been set forth in order to provide a thorough understanding of the embodiments. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects, and that one or more features or specific details from one embodiment may be practiced together with one or more features or specific details from another embodiment, where appropriate, in the practice of the disclosure.

## Claims

1. An electromagnetically heated scented candle (3), **characterized by**:
a container (31) including a bottom wall (311) and a surrounding wall (312) extending upwardly from a periphery of said bottom wall (311), said bottom wall (311) and said surrounding wall (312) cooperatively defining a receiving space (313), said bottom wall (311) being magnetically conductive; and
a scented candle body (32) received in said receiving space (313).

2. The electromagnetically heated scented candle as claimed in Claim 1, **characterized in that** said bottom wall (311) of said container (31) has a bottom surface (3111), and a magnetically conductive heating member (4) disposed on said bottom surface (3111) and located outside of said receiving space (313), said bottom surface (3111) having a thickness equal to or different from that of said surrounding wall (312).

3. The electromagnetically heated scented candle as claimed in Claim 2, further **characterized by** a thermally conductive bonding layer (5) disposed between said bottom surface (3111) of said container (31) and said magnetically conductive heating member (4), said thermally conductive bonding layer (5) being deformable.

4. A scented candle device, **characterized by**:
an electromagnetic base (2) including a casing (21) that defines a base space (22) and that has a top surface (211), and an electromagnetic unit (23) disposed in said base space (22) for generating a magnetic field;
an electromagnetically heated scented candle (3) disposed on said top surface (211), and including
a container (31) including a bottom wall (311) contactable with said top surface (211) of said casing (21), and a surrounding wall (312) extending upwardly from a periphery of said bottom wall (311), said bottom wall (311) and said surrounding wall (312) cooperatively defining a receiving space (313), and
a scented candle body (32) received in said receiving space (313); and
a magnetically conductive heating member (4) disposed between said top surface (211) of said casing (21) and said container (31), said magnetically conductive heating member (4) generating heat energy after interacting with the magnetic field of said electromagnetic unit (23) for heating said scented candle body (32).

5. The scented candle device as claimed in Claim 4, **characterized in that** said bottom wall (311) of said container (31) serves as a magnetically conductive heating member.

6. The scented candle device as claimed in Claim 4 or Claim 5, **characterized in that** said magnetically conductive heating member (4) is a magnetic spacer.

7. The scented candle device as claimed in any one of Claims 4 to 6, **characterized in that** said bottom wall (311) of said container (31) has a bottom surface (3111), said magnetically conductive heating member (4) being disposed on said bottom surface (3111) and located outside of said receiving space (313).

8. The scented candle device as claimed in any one of Claims 4 to 7, **characterized in that** said electromagnetic unit (23) includes a power supply (231), a control circuit (232) electrically connected to said power supply (231), and a loading coil (233) electrically connected to said control circuit (232), said loading coil (233) being capable of generating the magnetic field by current supplied by said power supply (231).

9. The scented candle device as claimed in any one of Claims 4 to 8, further **characterized by** a thermally conductive bonding layer (5) disposed between said bottom wall (311) of said container (31) and said magnetically conductive heating member (4), said thermally conductive bonding layer (5) being deformable.
